# EUROPEAN PATENT APPLICATION

(11) **EP 4 095 788 A1**
(43) Date of publication of application: **30.11.2022**
(21) Application number: 21744805.9
(22) Date of filing: 08.01.2021
(51) Int. Cl.: G06T 1/00, A61B 5/1172

(54) **SENSING DEVICE, METHOD, AND PROGRAM**

(30) Priority: 20.01.2020 JP 2020006844
(71) Applicant: Sony Group Corporation, Minato-Ku, Tokyo, 108-0075 (JP)
(72) Inventor: KAMADA, Yasunori, Tokyo 108-0075 (JP); SHIGEI, Hiroyuki, Tokyo 108-0075 (JP); NEGISHI, Atsushi, Tokyo 108-0075 (JP); ISHII, Tamotsu, Tokyo 108-0075 (JP); SUZUKI, Kenji, Tokyo 108-0075 (JP); OGATA, Takashi, Tokyo 108-0075 (JP)
(74) Representative: MFG Patentanwälte Meyer-Wildhagen Meggle-Freund Gerhard PartG mbB
(86) International application number: PCT/JP2021/000483
(87) International publication number: WO 2021/149515

(57) **Abstract**

A sensing device of an embodiment includes: a first sensor that acquires three-dimensional shape information on a surface of an object to be sensed; and a second sensor that acquires texture information on the surface or a vicinity of the surface of the object to be sensed at a position having a predetermined positional relation with an information acquisition position in the object to be sensed of the first sensor.

## Description

### Field

The present disclosure relates to a sensing device, a method, and a program.

### Background

In a common fingerprint authentication device, authentication is performed by using the shape of a ridge of a fingerprint (hereinafter, referred to as level 1 feature) and a branch and an end point of the ridge called minutiae as feature amounts (hereinafter, referred to as level 2 feature).

Level 2 authentication in which authentication is performed by using the level 2 feature needs a resolution of approximately 500 ppi. A common fingerprint sensor outputs binarized ridge information (fingerprint unevenness information) of approximately 500 ppi.

Furthermore, a fingerprint authentication device such as a smartphone adopts a registration phase and a collation phase. In the registration phase, a fingerprint of a person who is permitted to log in is registered. In the collation phase, the fingerprint is collated at the time of actual login.

In order to successfully perform collation in the collation phase, a part of a finger included in a registered area is required to be put on a sensor. Since a position at which a finger is put is shifted in collation operation in an actual environment, it is desirable to register a wide area without omission in the registration phase. In order to achieve such registration, in the registration phase, a user is commonly required to perform operation of putting a finger on a sensor while moving the finger many times. At this time, some devices determine an unregistered area, and provide feed back about a part of a finger which is to touch the sensor to the user through a user interface.

A feature of data registered so far is used to determine an unregistered area. Signal processing called stitching, in which a mutual positional relation (including rotation) is estimated, is sometimes used.

### Citation List

### Patent Literature

Patent Literature 1: JP 2017-196319 A

### Summary

### Technical Problem

Incidentally, sensors with improved authentication accuracy, which uses texture information of a finger (hereinafter, referred to as level 3 feature), have recently been started to be unveiled. Level 3 authentication that performs authentication by using a level 3 feature needs multivalued images of a resolution of several thousands of ppi. Also in such a sensor that acquires the level 3 feature, similarly to a sensor that acquires the level 2 feature, a part of a finger included in a registered area needs to be put on the sensor at the time of collation.

Since, however, an imaging element (CMOS sensor) is used as a sensor device for acquiring the level 3 feature, it is difficult to widen a sensing range as compared with in an electrostatic capacitive type and other types. Therefore, in order to register a wide area without omission in the registration phase, a larger number of trials are required, which causes a concern over a greater burden on a user.

Therefore, registration processing and authentication processing in which a burden on a user and an operation cost are inhibited have been desired.

The present technology has been made in view of such a situation, and an object thereof is to provide a sensing device, a method, and a program capable of performing registration processing and authentication processing in which a burden on a user and an operation cost are inhibited.

### Solution to Problem

A sensing device of the embodiment includes: a first sensor that acquires three-dimensional shape information on a surface of an object to be sensed; and a second sensor that acquires texture information on the surface or a vicinity of the surface of the object to be sensed at a position having a predetermined positional relation with an information acquisition position in the object to be sensed of the first sensor.

### Brief Description of Drawings

FIG. 1 is a cross-sectional view of one example of a sensing device of an embodiment.
FIG. 2 is an external perspective view of the sensing device in FIG. 1.
FIG. 3A is an explanatory view illustrating information acquisition performed by a stacked type sensor in FIG. 1.
FIG. 3B is an explanatory view illustrating the information acquisition performed by the stacked type sensor in FIG. 1.
FIG. 3C is an explanatory view illustrating the information acquisition performed by the stacked type sensor in FIG. 1.
FIG. 4A is an explanatory view illustrating operation at the time of registering fingerprint information.
FIG. 4B is an explanatory view illustrating the operation at the time of registering fingerprint information.
FIG. 5 is an external perspective view of an electronic device to which a sensing device of a first embodiment is applied.
FIG. 6 is an explanatory diagram of a schematic system configuration example of the electronic device.
FIG. 7 is an explanatory diagram of another schematic system configuration example of the electronic device.
FIG. 8 is a flowchart illustrating operation at the time of registering fingerprint data in the first embodiment.
FIG. 9 is a flowchart illustrating operation at the time of fingerprint authentication in a second embodiment.
FIG. 10 is an explanatory diagram of one example of a hierarchical database applied to a second variation of the second embodiment.
FIG. 11 is a flowchart illustrating operation at the time of fingerprint authentication in the second variation of the second embodiment.
FIG. 12 is an explanatory diagram of one example of a processing sequence chart of the second variation of the second embodiment.
FIG. 13 is an explanatory diagram of one example of a processing sequence chart of a third variation of the second embodiment.
FIG. 14 is a processing flowchart of a third embodiment.
FIG. 15 is a processing flowchart of a first variation of the third embodiment.
FIG. 16 is an explanatory diagram of a schematic system configuration example of a second variation of the third embodiment.
FIG. 17 is a processing flowchart of the second variation of the third embodiment.
FIG. 18 is a processing flowchart of a fourth embodiment.
FIG. 19 is a processing flowchart of a first variation of the fourth embodiment.
FIG. 20A is an explanatory view of a variation of an embodiment.
FIG. 20B is an explanatory view of a variation of the embodiment.
FIG. 20C is an explanatory view of a variation of the embodiment.

### Description of Embodiments

Embodiments will be described in detail below with reference to the drawings.

First, prior to the description of the embodiments, the principle of the embodiments will be described.

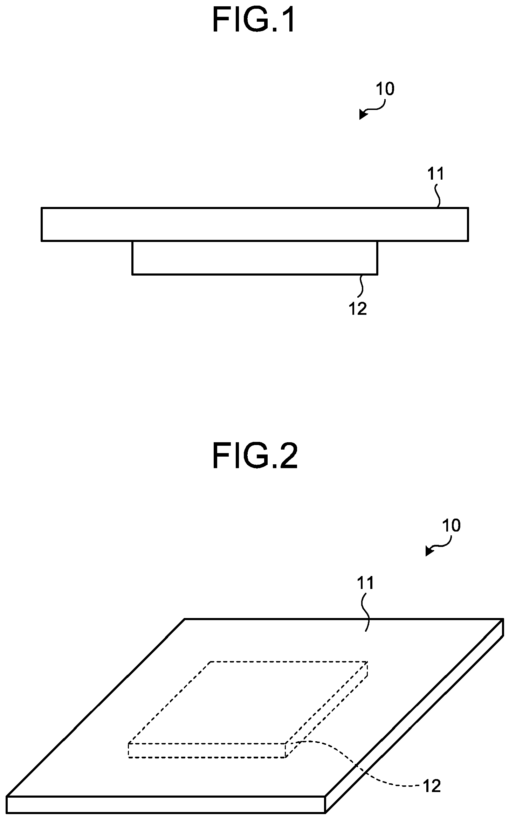

FIG. 1 is a cross-sectional view of one example of a sensing device of an embodiment.

FIG. 2 is an external perspective view of the sensing device in FIG. 1.

A sensing device 10 of the embodiment includes a first sensor 11 and a second sensor 12. The first sensor 11 adopts an electrostatic capacitive type, and has light transparency. The second sensor 12 adopts an imaging type.

In this case, the first sensor 11 acquires three-dimensional shape information on the surface of an object to be sensed.

Furthermore, the second sensor 12 acquires texture information on the surface or the vicinity of the surface of an object to be sensed at a position having a predetermined positional relation with an information acquisition position of an object to be sensed by the first sensor 11.

Examples of the object to be sensed by the sensing device 10 include a tissue on the surface or the vicinity of the surface of each part of a human body or a living body (e.g., dermis and blood vessel (such as vein)). Here, the human body and the living body include the surface of each part of a hand such as a finger (thumb [first finger], index finger [second finger], middle finger, digitus anularis [fourth finger], and little finger), a palm (thenar eminence, hypothenar eminence, and carpus), and the back of the hand.

Moreover, the three-dimensional shape information includes information such as a fingerprint.

Furthermore, the texture information on the surface of the object to be sensed includes information on the visual color and luminance, a pattern, arrangement, and the like of a tissue in the vicinity of the surface, such as the surface shape of the surface or a dermis of each part of a human body or a living body and a flow of a blood vessel. Specifically, the texture information includes an image of a fingerprint, skin, a blood vessel, and the like, information such as color of a skin, a dermis, a blood vessel, and the like, and information on an arrangement of a flow of a blood vessel and the like.

Description will be given below by taking a case where fingerprint authentication is performed for an example.

Incidentally, the fingerprint authentication is performed by using a level 1 feature, a level 2 feature, a level 3 feature, and the like. The level 1 feature relates to, for example, the flow of ridges of a fingerprint, such as a whorl pattern and an arch pattern. The level 2 feature relates to, for example, ridges, end points, and branch points of a thinned fingerprint. The level 3 feature relates to, for example, a sweat pore in the fingerprint invisible to the naked eyes and the thickness of a ridge.

The first sensor 11 performs fingerprint authentication by using the level 2 information among these pieces of information. The second sensor 12 performs fingerprint authentication by using the level 3 information thereamong.

The sensing device 10 in FIGS. 1 and 2 has a first size and a second size below the first sensor 11 of an electrostatic capacitive type. The first sensor 11 uses the level 2 feature, and has (detection light; visible light) transparency. The second size is smaller than the first size. The sensing device 10 is configured as a stacked type sensor obtained by stacking the second sensor 12 of an imaging type using the level 3 feature.

Although a case where visible light is used as detection light of the second sensor 12 will be described below, any detection light can be used in accordance with an imageable wavelength (any detection light including invisible light and the like such as infrared light and ultraviolet light in addition to visible light) of the second sensor 12.

In this case, the first sensor 11 has an installation area larger than the installation area of the second sensor 12, and constitutes a stacked type sensor in which the first sensor 11 is installed so as to cover the entire upper surface of the second sensor 12.

Furthermore, the second sensor 12 includes a close-up lens configured as a microlens array (MLA) that collects visible light transmitted through the first sensor 11.

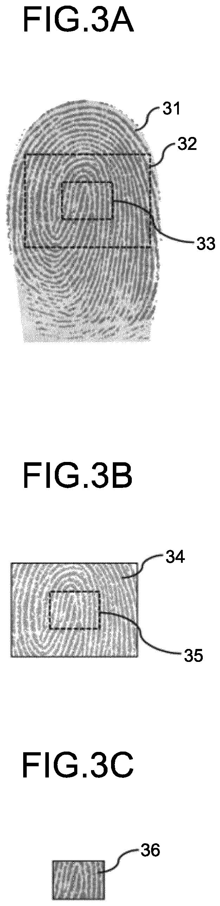

FIGS. 3A to 3C are explanatory views illustrating information acquisition performed by the stacked type sensor in FIG. 1.

FIG. 3A is an explanatory view illustrating the arrangement relation between an information acquisition region 32 of the first sensor 11 and an information acquisition region 33 of the second sensor with respect to a finger 31.

As illustrated in FIG. 3A, the information acquisition region 32 of the first sensor is set to overlap the information acquisition region 33 of the second sensor, and the information acquisition region 32 of the first sensor 11 is set to include the information acquisition region 33 of the second sensor 12.

Therefore, an image can be acquired such that an image acquired by the second sensor 12 of an imaging type corresponds to an area of an image acquired by the first sensor 11 of an electrostatic capacitive type with transparency.

FIG. 3B is an explanatory view obtained by visualizing information acquired by the first sensor 11.

As illustrated in FIG. 3B, an information acquisition region 34 of the first sensor 11 includes information such as ridges, end points, and branch points of a thinned fingerprint of the finger 31.

FIG. 3C is an explanatory view of acquired information of the second sensor 12.

As illustrated in FIG. 3C, image information is acquired in an information acquisition region 35 of the second sensor 12. The image information includes information such as a sweat pore in a fingerprint of the finger 31 invisible to the eyes and the thickness of a ridge.

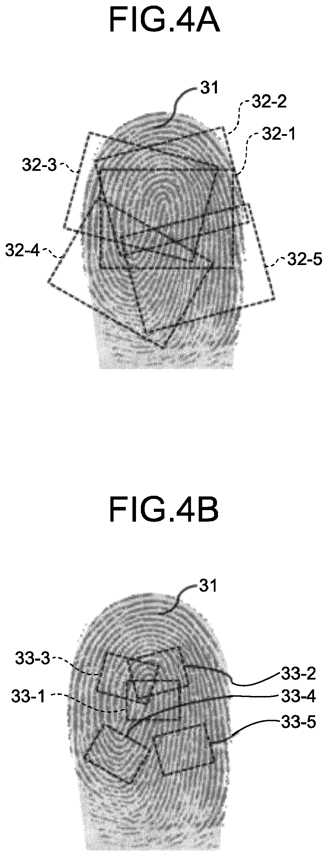

FIGS. 4A and 4B are explanatory views illustrating operation at the time of registering fingerprint information.

For example, assuming that information is acquired, for example, five times for the finger 31 at different information acquisition places, information of almost the entire fingerprint acquisition region of a finger can be acquired as illustrated in FIG. 4A, for example.

More specifically, information of the entire region necessary for fingerprint authentication can be obtained by the information collected in five information acquisition regions of information acquisition regions 32-1 to 32-5 of the first sensor.

In contrast, as illustrated in FIG. 4B, although the information acquisition regions 33-1 to 33-3 of the second sensor 12 partially overlap each other, the information acquisition region 33-4 and the information acquisition region 33-5 of the second sensor 12 do not overlap the other information acquisition regions. The arrangement relation of the information acquisition region 33-4 and the information acquisition region 33-5 with the other information acquisition regions 33-1 to 33-3 cannot be grasped.

Therefore, in this case, it is necessary to set an information acquisition region overlapping between the information acquisition regions corresponding to the information acquisition regions 33-1 to 33-3 of the second sensor 12 and the information acquisition region 33-4 or the information acquisition region 33-5 of the second sensor 12.

Such configuration can promote efficiency of operation in a registration phase in a level 3 authentication without increasing an operation cost. Reduction in the number of trials at the time of registration reduces a user load while eliminating an unregistered region. Authentication processing can be reliably performed.

Next, a more specific embodiment will be described.

### [1] First Embodiment

Next, a first embodiment will be described.

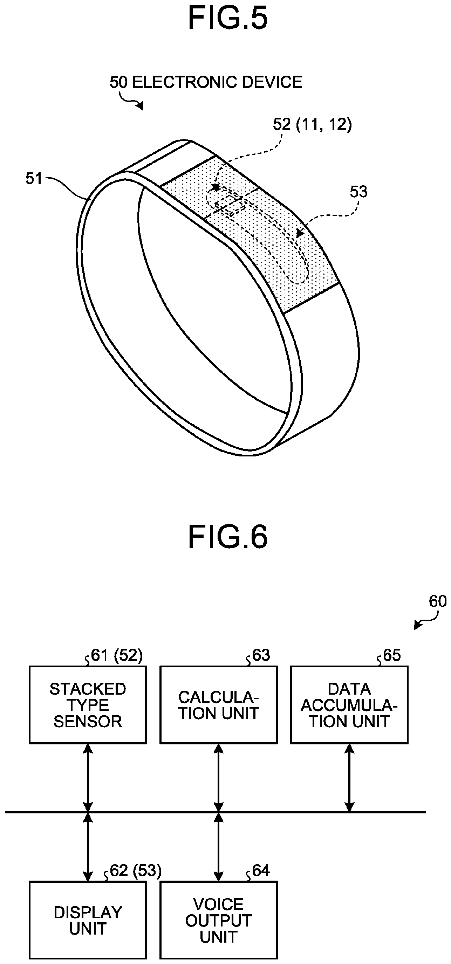

FIG. 5 is an external perspective view of an electronic device to which a sensing device of the first embodiment is applied.

An electronic device 50 includes a ring-shaped wristband main body 51, a sensing device (stacked type sensor) 52, and a display unit 53 that displays various pieces of information.

Here, the sensing device 52 and the display unit 53 are provided in a part of the wristband main body 51.

Furthermore, the sensing device 52 constitutes a stacked type sensor in which the first sensor 11 of an electrostatic capacitive type and the second sensor 12 of an imaging type are stacked as illustrated in FIGS. 1 and 2.

Moreover, the sensing device 52 can be provided in the wristband main body 51 side by side with the display unit 53. When the display unit 53 has transparency and has little influence on detection of an electrostatic capacitive type, the sensing device 52 can be provided in a stacked manner below the display unit 53.

FIG. 6 is an explanatory diagram of a schematic system configuration example of the electronic device.

A system 60 of the electronic device 50 includes a stacked type sensor 61, a display unit 62, a voice output unit 64, a calculation unit 63, and a data accumulation unit 65. The stacked type sensor 61 acquires fingerprint data. The display unit 62 provides a user interface. The calculation unit 63 performs system control processing and various pieces of algorithm calculation processing. The data accumulation unit 65 holds a program and data related to fingerprint authentication.

In the above configuration, when registering (acquiring) fingerprint data, a user of the electronic device 50 presses a finger whose fingerprint data is to be registered against the stacked type sensor 61 based on guide information displayed on the display unit 62 under the control of the calculation unit 63, guide voice output by the voice output unit 64, and the like.

This causes the calculation unit 63 to collect fingerprint data and accumulate the fingerprint data in the data accumulation unit 65 based on output of the stacked type sensor 61, and use the fingerprint data at the time of subsequent fingerprint authentications.

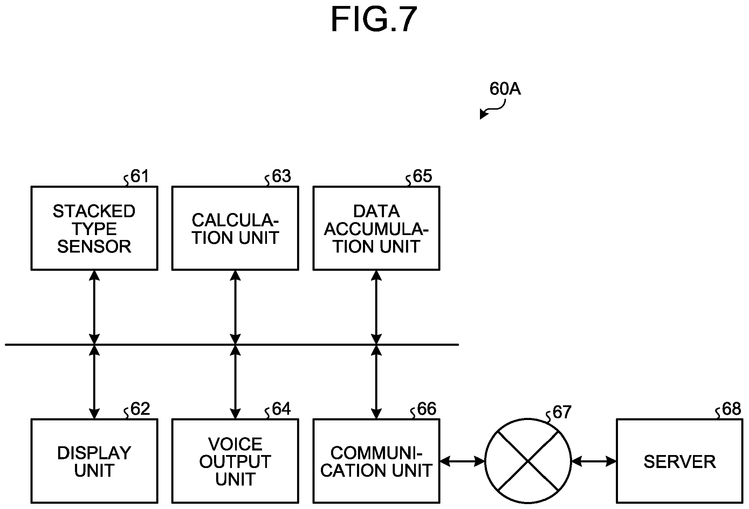

FIG. 7 is an explanatory diagram of another schematic system configuration example of the electronic device.

A system 60A of the electronic device 50 includes the stacked type sensor 61, the display unit 62, the voice output unit 64, the calculation unit 63, the data accumulation unit 65, and a communication unit 66. The stacked type sensor 61 acquires fingerprint data. The display unit 62 provides a user interface. The calculation unit 63 performs system control processing and various pieces of algorithm calculation processing. The data accumulation unit 65 holds a program and data related to fingerprint authentication.

In the above-described configuration, when registering (acquiring) fingerprint data, the user of the electronic device 50 presses a finger whose fingerprint data is to be registered against the stacked type sensor 61 based on guide information displayed on the display unit 62 under the control of the calculation unit 63, guide voice output by the voice output unit 64, and the like. Thus, the calculation unit 63 collects fingerprint data and accumulates the fingerprint data in the data accumulation unit 65 based on output of the stacked type sensor 61 while registering the fingerprint data in a server 68 via the communication unit 66 and a communication network 67.

At the time of fingerprint authentication after the fingerprint data is registered, the calculation unit 63 acquires the fingerprint data based on the output of the stacked type sensor 61, and requests the server 68 to perform the fingerprint authentication via the communication unit 66 and the communication network 67.

This causes the server 68 to perform the fingerprint authentication based on the registered fingerprint data and the fingerprint data corresponding to the fingerprint authentication request and notify the calculation unit 63 of the fingerprint authentication result via the communication network 67 and the communication unit 66.

This causes the calculation unit 63 to perform subsequent processing based on the given fingerprint authentication result.

For example, shift to various pieces of processing requested by the user or notification of information indicating that failure of fingerprint authentication prevents the shift to various pieces of requested processing may be performed

Next, the operation of the first embodiment will be described in detail.

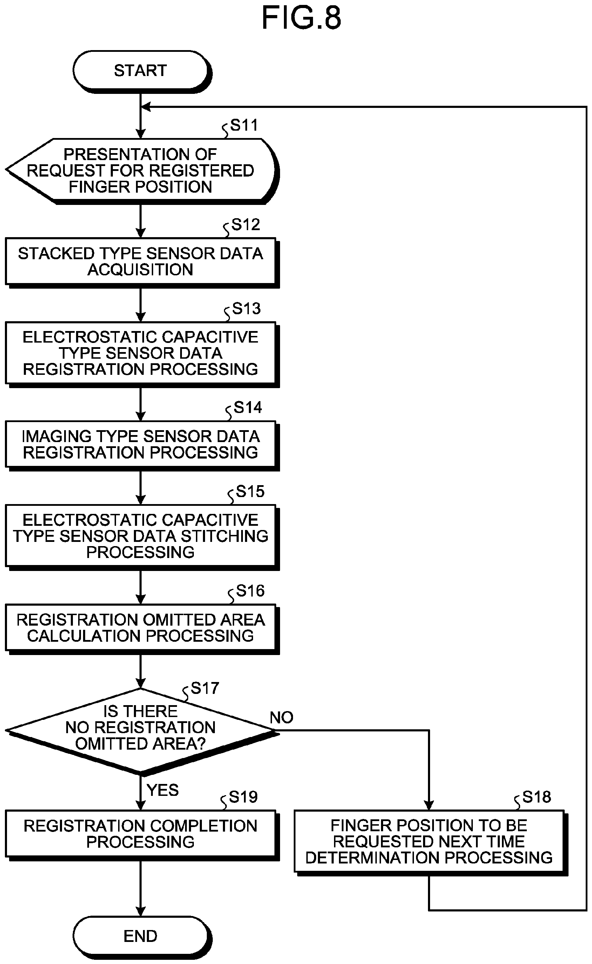

FIG. 8 is a flowchart illustrating operation at the time of registering fingerprint data in the first embodiment.

First, the calculation unit 63 performs processing of presenting a finger position for fingerprint registration to a user by using the display unit 62 and the voice output unit 64 (Step S11).

Presentation of the finger position which a user is requested to perform for fingerprint registration may be performed by displaying a detailed coordinate-based instruction by displaying a GUI on the display unit 62 or outputting an abstract instruction such as "upper part/lower part/right/left of finger" and "central part/peripheral part of finger" with the voice output unit 64.

Alternatively, a vibrator may be incorporated to promote movement of a finger by vibration. Furthermore, an indicator representing a finger moving direction, such as an LED, may be arranged around the display unit.

Furthermore, a predetermined finger position or a random finger position may be displayed as a registered finger position immediately after the start of registration of fingerprint data.

Next, the calculation unit 63 acquires data of the first sensor 11 of an electrostatic capacitive type and the second sensor 12 of an imaging type, which constitute the stacked type sensor 61 (Step S12).

Next, the calculation unit 63 registers the data of the first sensor 11 acquired in Step S12 in the data accumulation unit 65 as first fingerprint data (Step S13).

Similarly, the calculation unit 63 registers the data of the second sensor 12 acquired in Step S12 in the data accumulation unit 65 as second fingerprint data (Step S14).

Although, in the above description, the first fingerprint data of the first sensor 11 and the second fingerprint data of the second sensor 12, which have been acquired, are registered in the data accumulation unit 65, configuration in which the fingerprint data is registered in the server 68 can be used when the configuration in FIG. 7 is adopted.

Furthermore, there can be used configuration in which a feature amount for authentication is extracted by offload processing in the calculation unit 63 and the server 68 for the first fingerprint data and the second fingerprint data, which have been acquired, and fingerprint feature amount data corresponding to the extracted feature amount is stored in the data accumulation unit 65 and the server 68.

Moreover, the fingerprint data or the fingerprint feature amount data may be registered in the data accumulation unit 65 after being encrypted.

Furthermore, the first fingerprint data acquired by the first sensor 11 of an electrostatic capacitive type may be used only for registration processing in an interlocking (stitching) use for grasping the positional relation between pieces of fingerprint data. When only the second fingerprint data acquired by the second sensor 12 of an imaging type is used for an authentication use, registration processing in the authentication use can be omitted.

Subsequently, the calculation unit 63 performs stitching processing of calculating a mutual positional relation between a first fingerprint data group and the first fingerprint data acquired this time (Step S15). The first fingerprint data group is an aggregation of a plurality of pieces of acquired first fingerprint data corresponding to the first sensor 11 of an electrostatic capacitive type.

When the stitching processing is performed, a certain number of pieces of data for grasping the mutual positional relation needs to be accumulated to calculate the positional relation. Therefore, the processing in Step S15 can be skipped from the start of acquisition of fingerprint data to accumulation of data that can be calculated in the stitching processing.

Furthermore, the second fingerprint data acquired by the second sensor 12 of an imaging type may be supplementarily used as information used in the stitching. In this case, fine adjustment of a position and rotation, correction of finger distortion that occurs at the time when the finger is pressed, and the like using a high resolution are assumed as a method of using the fingerprint data acquired by the second sensor 12 of an imaging type.

Next, the calculation unit 63 calculates a registration omitted area by using information obtained by the stitching processing in Step S15.

For example, in order to clarify the arrangement relation of the information acquisition region 33-4 and the information acquisition region 33-5 in FIG. 4B with the information acquisition regions 33-1 to 33-3 and the like, and integrally address these information acquisition regions, a region between a first region represented by the information acquisition regions 33-1 to 33-3 and a second region represented by the information acquisition region 33-4 and the information acquisition region 33-5 is required to be set as a registration omitted area.

This allows the information acquisition regions 33-1 to 33-5 to be addressed as an integrated region, and allows the mutual arrangement relation to be clarified if the second fingerprint data corresponding to the registration omitted area can be acquired. Moreover, the registration omitted area may be an area that has never been registered or area for which a heat map based on the number of times of registration is generated and the number of times of registration does not satisfy a standard.

Next, the calculation unit 63 determines whether there is no registration omitted area (Step S17).

If it is determined in Step S17 that there is no registration omitted area (Step S17; Yes), registration completion processing for the obtained first fingerprint data and second fingerprint data is performed, and the processing ends (Step S19).

When it is determined in Step S17 that there is still a registration omitted area as illustrated in FIG. 4B, a finger position for which a request is given to a user next time (finger position to be requested next time) for locating the second sensor 12 in the registration omitted area is determined (Step S18), and the processing proceeds to Step S11 again.

In this case, in principle, the finger position to be requested next time is determined based on the information on the registration omitted area calculated by the calculation unit 63 in Step S16. A predetermined finger position, however, may be output as the finger position to be requested next time, or a random finger position may be output as the finger position to be requested next time until a sufficient number of pieces of fingerprint data for performing the stitching processing in Step S15 is accumulated.

As a result, the finger position to be requested next time determined in Step S18 is presented to the user in the processing in Step S11.

The stitching processing can be performed by performing the above-described processing. The second fingerprint data with which a mutual positional relation can be grasped can be obtained, and used in the fingerprint authentication.

One set of pieces of fingerprint authentication data (plurality of pieces of second fingerprint data subjected to at least stitching processing) is acquired in the above description. When fingerprint authentication data for a plurality of fingers is acquired, the above-described processing is repeated a plurality of times.

### [2] Second Embodiment

Next, a second embodiment will be described.

Although, in the first embodiment, a configuration in which the second fingerprint data subjected to at least the stitching processing is acquired for performing fingerprint authentication by using the second fingerprint data acquired by the second sensor 12 has been described, the second embodiment describes efficient fingerprint authentication using the first fingerprint data acquired by the first sensor 11 and the second fingerprint data acquired by the second sensor 12.

As described above, according to the first sensor 11 of an electrostatic capacitive type, ridge information serving as the level 1 feature and minutiae information serving as the level 2 feature can be acquired.

In contrast, texture information serving as the level 3 feature can be acquired by the second sensor 12 of an imaging type.

Therefore, the level 1 feature to the level 3 feature can be simultaneously acquired. In the second embodiment, efficient fingerprint authentication is performed by using a combination thereof.

As a result, an effect of increasing authentication accuracy can be obtained as compared with the case where each level feature is individually used.

Specifically, since the second sensor 12 of an imaging type has a narrow sensing range, the level 3 feature acquired by the second sensor 12 may cause other person acceptance in which another person who is locally very similar to a person himself/herself is authenticated as the person himself/herself. In such a case, the other person acceptance rate can be reduced and authentication accuracy can be improved by combining the level 1 feature and the level 2 feature with the level 3 feature. The level 1 feature and the level 2 feature have been acquired in a wide range acquired by the first sensor 11 of an electrostatic capacitive type having a wide sensing range.

Since a device and a system configuration applied to the second embodiment are similar to those of the first embodiment, those will be referred to. Fingerprint authentication operation of the second embodiment will be described below on the assumption that fingerprint authentication data has already been registered.

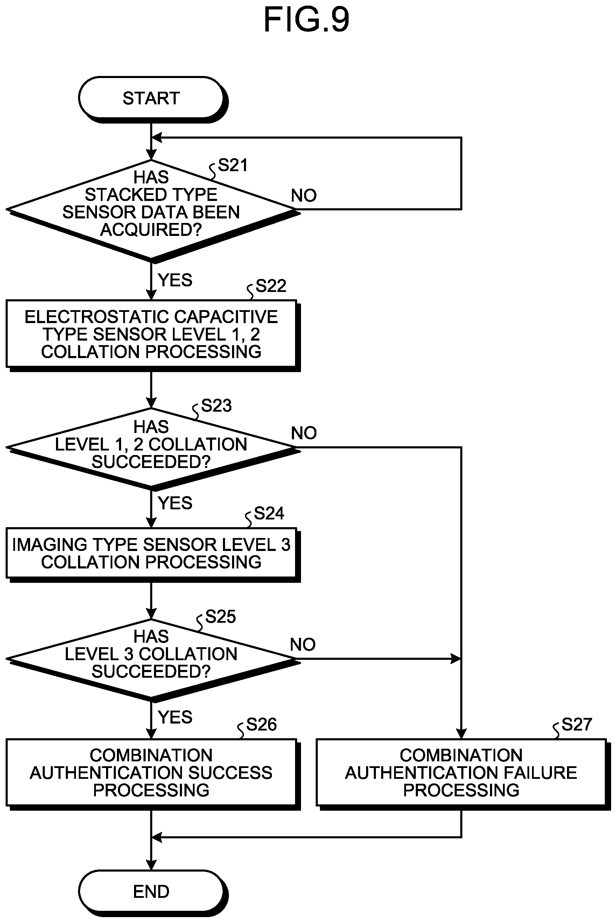

FIG. 9 is a flowchart illustrating operation at the time of fingerprint authentication in the second embodiment.

First, the calculation unit 63 acquires the first fingerprint data from the first sensor 11 of an electrostatic capacitive type, which constitutes the stacked type sensor 61, and determines whether or not the second fingerprint data has been acquired from the second sensor 12 of an imaging type (Step S21).

When it is determined in Step S21 that the first fingerprint data and the second fingerprint data necessary for the processing have not been acquired yet (Step S21; No), a standby state is set.

When it is determined in Step S21 that the first fingerprint data of the first sensor 11 of an electrostatic capacitive type necessary for the processing has been acquired and the second fingerprint data of the second sensor 12 of an imaging type has been acquired (Step S21; Yes), the calculation unit 63 performs collation processing on the level 1 feature and the level 2 feature based on the first fingerprint data acquired by the first sensor 11 (Step S23).

Specifically, similarities of the level 1 feature and the level 2 feature is calculated with reference to the first fingerprint data for fingerprint authentication registered in the data accumulation unit 65.

Subsequently, the calculation unit 63 determines whether or not the similarities of the level 1 feature and the level 2 feature obtained with respect to the first fingerprint data acquired this time exceeds a predetermined threshold, that is, whether or not the collation of the level 1 feature, the collation of the level 2 feature, and the collation of the combination of the level 1 feature and the level 2 feature have succeeded (Step S23).

When it is determined in Step S23 that the collation of the level 1 feature, the collation of the level 2 feature, and the collation of the combination of the level 1 feature and the level 2 feature have failed (Step S23; No), the processing proceeds to Step S27.

When it is determined in Step S23 that the collation of the level 1 feature, the collation of the level 2 feature, and the collation of the combination of the level 1 feature and the level 2 feature have succeeded (Step S23; Yes), the calculation unit 63 performs collation processing on the level 3 feature based on the second fingerprint data acquired by the second sensor 12 (Step S24).

Subsequently, the calculation unit 63 determines whether or not the similarity of the level 3 feature obtained with respect to the second fingerprint data acquired this time has exceeded a predetermined threshold, that is, whether or not the collation of the level 3 feature has succeeded (Step S25).

When it is determined in Step S25 that the collation of the level 3 feature has succeeded (Step S25; Yes), the calculation unit 63 determines that the combination authentication has succeeded, performs combination authentication success processing such as shifting to processing after the fingerprint authentication, and ends the processing.

In this case, possible processing after the fingerprint authentication includes, for example, issuance of some right or key to a user who has attempted authentication, performance of settlement at the time of shopping, and the like.

Moreover, the display unit 62 and the voice output unit 64 may perform feedback processing of presenting information of authentication success to the user.

When it is determined in Step S25 that the collation of the level 3 feature has failed (Step S25; No), the calculation unit 63 determines that the combination authentication has failed, performs combination authentication failure processing, and ends the processing (Step S27). The combination authentication failure processing includes notification of the information of the failure, prohibition of shift to the processing after the fingerprint authentication, and the like.

### First Variation of Second Embodiment

Next, a first variation of the second embodiment will be described.

Incidentally, it is known that sensors of the same type have a correlation between a size of a sensor and authentication accuracy, and decreasing the size of a sensor reduces a grasped feature amount and deteriorates the authentication accuracy.

In the second embodiment, the effects of improving the authentication accuracy by the combination collation of the feature amounts (level 1 feature to level 3 feature) have been described. According to the first variation of the second embodiment, however, the size (area) of a sensor can be reduced while the authentication accuracy is maintained by performing the combination collation of the feature amounts (level 1 feature to level 3 feature) instead of improving the authentication accuracy. An effect of reducing power consumption can be obtained by a sensor downsized by such configuration.

### Second Variation of Second Embodiment

Although, in the second embodiment, the combination collation of feature amounts has been described, a second variation of the second embodiment is an embodiment in which an operation cost at the time of collation is reduced by registering the feature amount with a small information amount acquired by the first sensor 11 of an electrostatic capacitive type and a feature amount with a large information amount acquired by the second sensor 12 of an imaging type as hierarchized databases.

For example, when one-to-N level 3 authentication is performed (more specifically, when collation with registration data of all citizens is performed to authenticate one individual), collation processing is required to be performed on a large amount of registration data.

A large information amount of the level 3 feature, however, causes a huge amount of operation cost if collation processing is tried to be performed on all the registration data.

Therefore, in the second variation of the second embodiment, collation processing using the level 1 feature and the level 2 feature is performed and candidate registration data is narrowed down in a preceding stage. The operation cost of level 3 collation processing is thereby reduced.

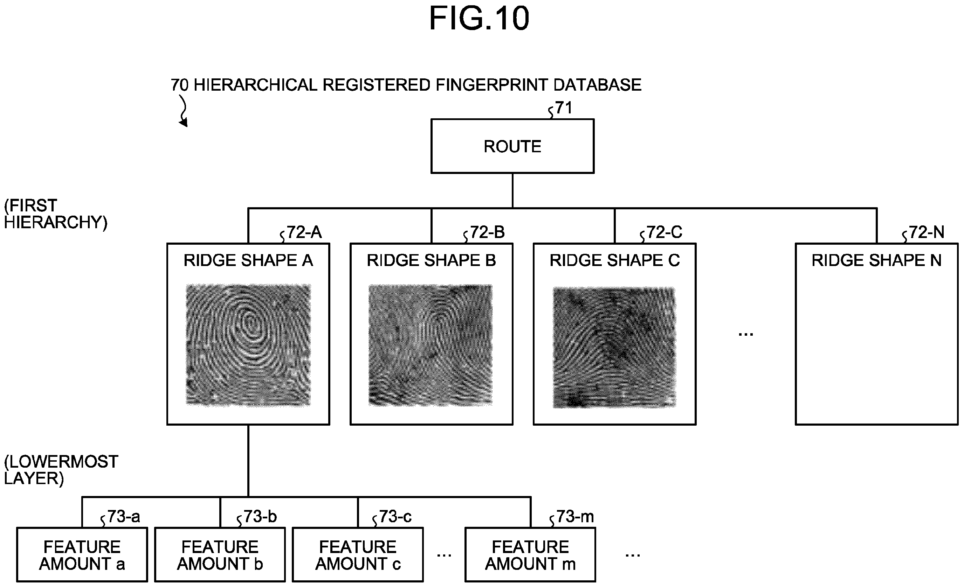

FIG. 10 is an explanatory diagram of one example of a hierarchical database applied to a second variation of the second embodiment.

A hierarchical registered fingerprint database 70 stores feature amount data 73-a, 73-b,..., and 73-m corresponding to feature amounts a, b,..., and m of the lowermost layer.

In the hierarchical registered fingerprint database 70, in order to improve search efficiency from a route 71, the feature amount data 73-a, 73-b,..., and 73-m corresponding to the respective feature amounts a, b,..., and m are preliminarily classified and stored in accordance with the level 1 feature acquired by the first sensor 11 of an electrostatic capacitive type.

Specifically, in a first hierarchy of the hierarchical registered fingerprint database 70, the feature amount data 73-a, 73-b,..., and 73-m corresponding to the respective feature amounts a, b,..., and m are preliminarily classified and stored in accordance with a ridge shape type which is the level 1 feature, that is, ridge shape data 72-A, 72-B,..., and 72-N corresponding to ridge shapes A, B,..., and N.

Although a case where the feature amount data 73-a, 73-b,..., and 73-m corresponding to the feature amounts a, b,..., and m of the lowermost layer are classified in accordance with the level 1 feature has been described above, the hierarchical registered fingerprint database 70 can be constructed by performing the classification by further using the level 2 feature (minutiae information).

Next, operation in the second variation of the second embodiment will be described.

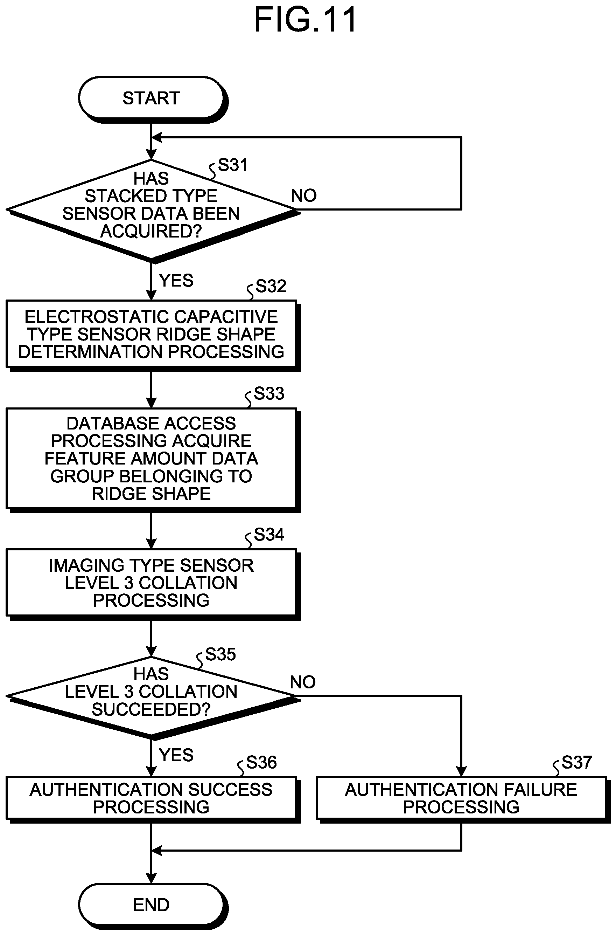

FIG. 11 is a flowchart illustrating operation at the time of fingerprint authentication in the second variation of the second embodiment.

First, the calculation unit 63 acquires the first fingerprint data from the first sensor 11 of an electrostatic capacitive type, which constitutes the stacked type sensor 61, and determines whether or not the second fingerprint data has been acquired from the second sensor 12 of an imaging type (Step S31).

When it is determined in Step S31 that the first fingerprint data and the second fingerprint data necessary for the processing have not been acquired yet (Step S31; No), a standby state is set.

When it is determined in Step S31 that the first fingerprint data of the first sensor 11 of an electrostatic capacitive type necessary for the processing has been acquired and the second fingerprint data of the second sensor 12 of an imaging type has been acquired (Step S31; Yes), the calculation unit 63 performs processing of determining a ridge shape, which is the level 1 feature, based on the first fingerprint data acquired by the first sensor 11 (Step S32).

Specifically, the calculation unit 63 calculates the similarity of a ridge shape based on the level 1 feature, determines the ridge shape, and determines which of the ridge shape data 72-A, 72-B,..., and 72-N corresponding to the ridge shapes A, B,..., and N the ridge shape belongs to with reference to the first fingerprint data for fingerprint authentication registered in the data accumulation unit 65.

Subsequently, the calculation unit 63 acquires a feature amount data group belonging to the ridge shape of the determination result from the data accumulation unit 65 (Step S33).

Subsequently, the calculation unit 63 accesses the hierarchical registered fingerprint database 70 constructed in the data accumulation unit 65, or accesses the hierarchical registered fingerprint database 70 constructed in the external server 68 through the communication network 67 by the communication unit 66.

In this case, the calculation unit 63 makes a data request by using the ridge shape type corresponding to the determination result in Step S32 as a key, and narrows down a corresponding registered feature amount data group as data to be collated in the level 3 collation processing.

In this case, when the hierarchical registered fingerprint database 70 is constructed in the external server 68, the registered feature amount data group preliminarily narrowed down by the communication unit 66 may be received and stored in the data accumulation unit 65.

Next, the calculation unit 63 performs processing of collation with the registered feature amount data group registered in the data accumulation unit 65 for the level 3 feature based on the second fingerprint data acquired by the second sensor 12 (Step S34).

Subsequently, the calculation unit 63 determines whether or not the similarity of the level 3 feature obtained with respect to the second fingerprint data acquired this time has exceeded a predetermined threshold, that is, whether or not the collation of the level 3 feature has succeeded (Step S35).

When it is determined in Step S35 that the collation of the level 3 feature has succeeded (Step S35; Yes), the calculation unit 63 determines that the combination authentication has succeeded, performs combination authentication success processing such as shifting to processing after the fingerprint authentication, and ends the processing as described above.

When it is determined in Step S35 that the collation of the level 3 feature has failed (Step S35; No), the calculation unit 63 determines that the combination authentication has failed, performs combination authentication failure processing, and ends the processing (Step S37). The combination authentication failure processing includes notification of the information of the failure, prohibition of shift to the processing after the fingerprint authentication, and the like.

Here, processing between a system and a database in the second variation of the second embodiment will be described.

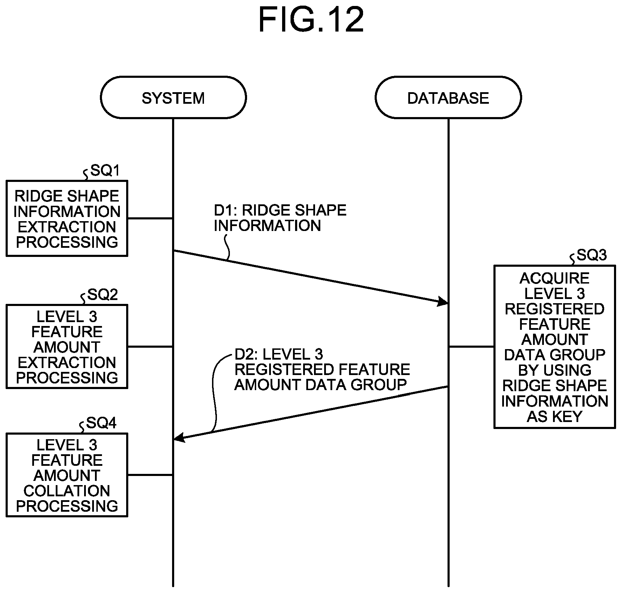

FIG. 12 is an explanatory diagram of one example of a processing sequence chart of the second variation of the second embodiment.

In FIG. 12, a system corresponds to the system 60 of the electronic device 50, and a database corresponds to the hierarchical registered fingerprint database 70 constructed in the data accumulation unit 65 or the server 68.

First, the system performs processing of extracting information on a ridge shape which is the level 1 feature acquired by the first sensor 11 of an electrostatic capacitive type, extracts ridge shape information D1 (Step SQ1), and outputs the ridge shape information D1 to a database.

This causes the database to acquire a level 3 feature amount data group registered by using ridge shape information D as a key (Step SQ3) and return an acquired level 3 registered feature amount data group D2 to the system.

In contrast, the system extracts the level 3 feature amount from fingerprint data acquired by the second sensor 12 of an imaging type (Step SQ2).

Moreover, when receiving the level 3 registered feature amount data group D2 from the database, the system performs level 3 feature amount collation on the acquired level 3 registered feature amount data group (Step SQ4), and obtains a collation result.

As illustrated above, according to the second variation of the second embodiment, collation processing using the level 1 feature and the level 2 feature is performed and candidate registration data is narrowed down in a preceding stage. The operation cost of the level 3 collation processing is thereby reduced, and the collation processing can be efficiently performed.

### Third Variation of Second Embodiment

In the above-described second variation of the second embodiment, a sensing device capable of reducing the operation cost of collation processing and the number of accesses of a database has been described as an example of relatively small-scale one-to-N level 3 authentication targeting a wearable device and the like having a limitation on the operation cost and power consumption. A third variation is an embodiment in which a relatively large-scale one-to-N level 3 authentication capable of processing a large amount of data in parallel on a server side is performed, and relates to speed-up of the authentication processing.

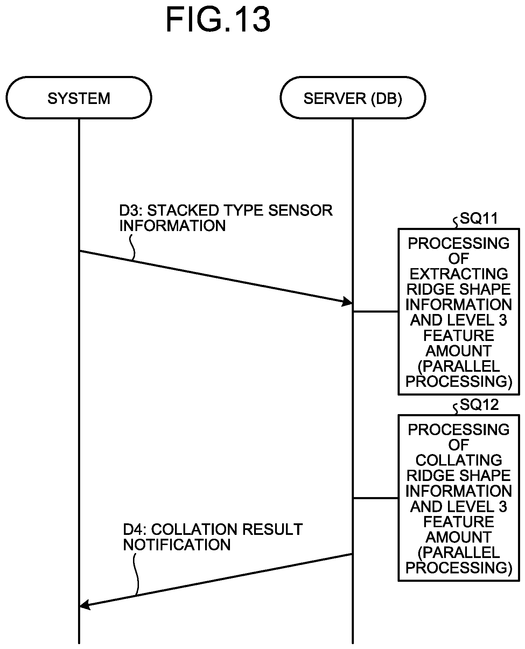

FIG. 13 is an explanatory diagram of one example of a processing sequence chart of the third variation of the second embodiment.

In FIG. 13, a system corresponds to the system 60 of the electronic device 50, and a server corresponds to the server 68.

First, the system outputs stacked type sensor information D3 to the server. The stacked type sensor information D3 includes both information (e.g., ridge shape information) of the first sensor 11 of an electrostatic capacitive type and information (e.g., level 3 feature amount) of the second sensor 12 of an imaging type. The stacked type sensor information D3 transmitted here may be subjected to encryption or compression processing on the system side, or may be subjected to some primary processing on the system side.

This causes the server to extract the ridge shape information and the level 3 feature amount in parallel (Step SQ11).

At this time, the server performs collation processing in parallel for the ridge shape data 72-A, 72-B,..., and 72-N corresponding to the ridge shapes A, B,..., and N of the first hierarchy in FIG. 10 in relation to the ridge shape information. The server performs collation processing in parallel for the feature amount data 73-a, 73-b,..., and 73-m corresponding to the feature amounts a, b,..., and m of the lowermost layer in relation to the level 3 feature amount (Step SQ12).

Then, the collation processing is interrupted at the time when a collation result is obtained. A collation result notification D4 is returned to the system.

As described above, according to the third variation of the second embodiment, time taken for obtaining a collation result can be averagely shorten by sequentially inputting data ready for processing to a parallel calculator.

### [3] Third Embodiment

Next, a third embodiment will be described.

In the third embodiment, the frequency of authentication rejection is reduced by adaptively switching and using the first sensor 11 of an electrostatic capacitive type and the second sensor 12 of an imaging type, which have different advantages and disadvantages, in accordance with an environment and a situation.

For example, in a case of a wet finger, the first sensor 11 of an electrostatic capacitive type may fail to acquire the first fingerprint data of a desired quality while the second sensor 12 of an imaging type is highly likely to be able to acquire the second fingerprint data of the desired quality due to excellent wet finger resistance of the second sensor 12.

In contrast, in a case of a dry finger or a finger smudged with ink and the like, the second sensor 12 of an imaging type may fail to acquire fingerprint data of a desired predetermined quality while the first sensor 11 of an electrostatic capacitive type is highly likely to be able to acquire the first fingerprint data of the desired quality due to excellent dry finger resistance and resistance to smudge on a finger surface of the first sensor 11.

Furthermore, in a case where the sensing device is used in an outdoor environment with strong sunlight, the second sensor 12 of an imaging type may fail to acquire the second fingerprint data of a desired quality while the first sensor 11 of an electrostatic capacitive type is highly likely to be able to acquire the first fingerprint data of the desired quality due to excellent ambient light resistance of the first sensor 11.

Incidentally, an authentication level of the first sensor 11 of an electrostatic capacitive type used in the sensing device of the third embodiment is the level 1 feature and the level 2 feature. An authentication level with the second sensor 12 of an imaging type is the level 3 feature. Therefore, although not all authentication levels can be necessarily addressed due to different achievable authentication levels, according to the third embodiment, a failure-to-acquire rate (FTA) can be reduced in a use case where a required authentication level is satisfied.

Since a device and a system configuration applied to the third embodiment are similar to those of the first embodiment, those will be referred to. Fingerprint authentication operation of the third embodiment will be described below on the assumption that fingerprint authentication data has already been registered.

Next, operation in the third embodiment will be described.

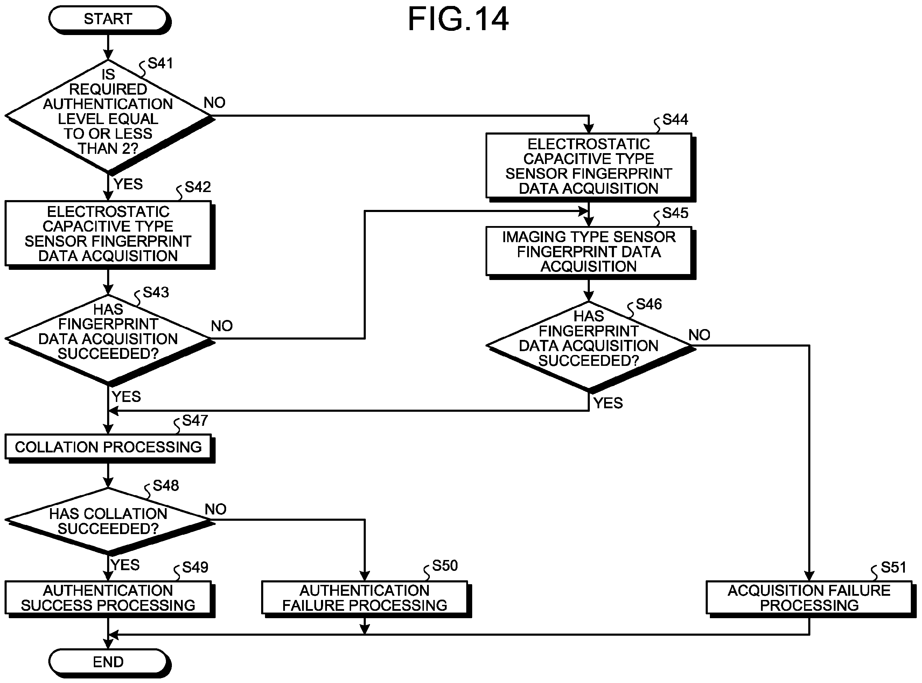

FIG. 14 is a processing flowchart of the third embodiment.

First, the calculation unit 63 determines an authentication level required for authentication of this time.

Specifically, the calculation unit 63 determines whether or not the required authentication level is equal to or less than the level 2 feature (Step S41).

When it is determined in Step S41 that the required authentication level is equal to or less than the level 2 feature (Step S41; Yes), the calculation unit 63 acquires the first fingerprint data from the first sensor 11 of an electrostatic capacitive type (Step S42).

Subsequently, the calculation unit 63 determines whether or not the first sensor 11 has succeeded in acquiring the first fingerprint data (Step S43).

When it is determined in Step S43 that the first sensor 11 has failed in acquiring the first fingerprint data (Step S43; No), the calculation unit 63 shifts the processing to Step S45.

When it is determined in Step S43 that the first sensor 11 has succeeded in acquiring the first fingerprint data (Step S43; Yes), the calculation unit 63 performs collation processing on the first fingerprint data acquired this time (Step S47).

Subsequently, the calculation unit 63 determines whether or not the collation processing in Step S47 has succeeded (Step S48).

When it is determined in Step S48 that the collation processing has succeeded (Step S47; Yes), the calculation unit 63 determines that the authentication has succeeded, performs authentication success processing such as shifting to processing after the fingerprint authentication, and ends the processing (Step S49).

In this case, possible processing after the fingerprint authentication includes, for example, issuance of some right or key to a user who has attempted authentication, performance of settlement at the time of shopping, and the like.

Moreover, the display unit 62 and the voice output unit 64 may perform feedback processing of presenting information of authentication success to the user.

When it is determined in Step S48 that the collation processing has failed (Step S48; No), the calculation unit 63 determines that the authentication has failed, performs authentication failure processing, and ends the processing (Step S50). The authentication failure processing includes notification of the information of the failure, prohibition of shift to the processing after the fingerprint authentication, and the like.

In contrast, when it is determined in Step S41 that the required authentication level exceeds the level 2 feature, that is, when the required authentication level is the level 3 feature, the calculation unit 63 first acquires the first fingerprint data from the first sensor 11 of an electrostatic capacitive type (Step S44).

Subsequently, the calculation unit 63 acquires the second fingerprint data from the second sensor 12 of an imaging type (Step S44) .

Subsequently, the calculation unit 63 determines whether or not the first sensor 11 and the second sensor 12 have respectively succeeded in requiring the first fingerprint data and the second fingerprint data (Step S46).

When it is determined in Step S46 that the first sensor 11 and the second sensor 12 have respectively failed in requiring the first fingerprint data and the second fingerprint data (Step S46; No), the calculation unit 63 performs the authentication failure processing such as notification of information indicating the failure of the authentication processing since the authentication processing has failed, and ends the processing (Step S51).

When it is determined in Step S46 that the first sensor 11 and the second sensor 12 have respectively succeeded in requiring the first fingerprint data and the second fingerprint data (Step S46; Yes), the calculation unit 63 performs collation processing on the first fingerprint data and the second fingerprint data acquired this time (Step S47).

Subsequently, the calculation unit 63 determines whether or not the collation processing in Step S47 has succeeded (Step S48).

When it is determined in Step S48 that the collation processing has succeeded (Step S47; Yes), the calculation unit 63 determines that the authentication has succeeded, performs authentication success processing such as shifting to processing after the fingerprint authentication, and ends the processing (Step S49).

When it is determined in Step S48 that the collation processing has failed (Step S48; No), the calculation unit 63 determines that the authentication has failed, performs authentication failure processing, and ends the processing (Step S50).

As described above, according to the third embodiment, in the case where the required authentication level is less than the level 2 feature, a failure-to-authenticate rate (FTA) can be reduced as compared with that of an electronic device including the first sensor 11 of an electrostatic capacitive type alone or the second sensor 12 of an imaging type alone.

### First Variation of Third Embodiment

Although, in the above description of the third embodiment, the required authentication level is assumed to be given from the outside, the first variation of the third embodiment is an embodiment in which the electronic device 50 preliminarily performs authentication processing and the authenticated state is maintained.

Since a device and a system configuration applied to the first variation of the third embodiment are similar to those of the first embodiment, those will be referred to. Fingerprint authentication operation of the first variation of the third embodiment will be described below on the assumption that fingerprint authentication data has already been registered.

Next, operation in the first variation of the third embodiment will be described.

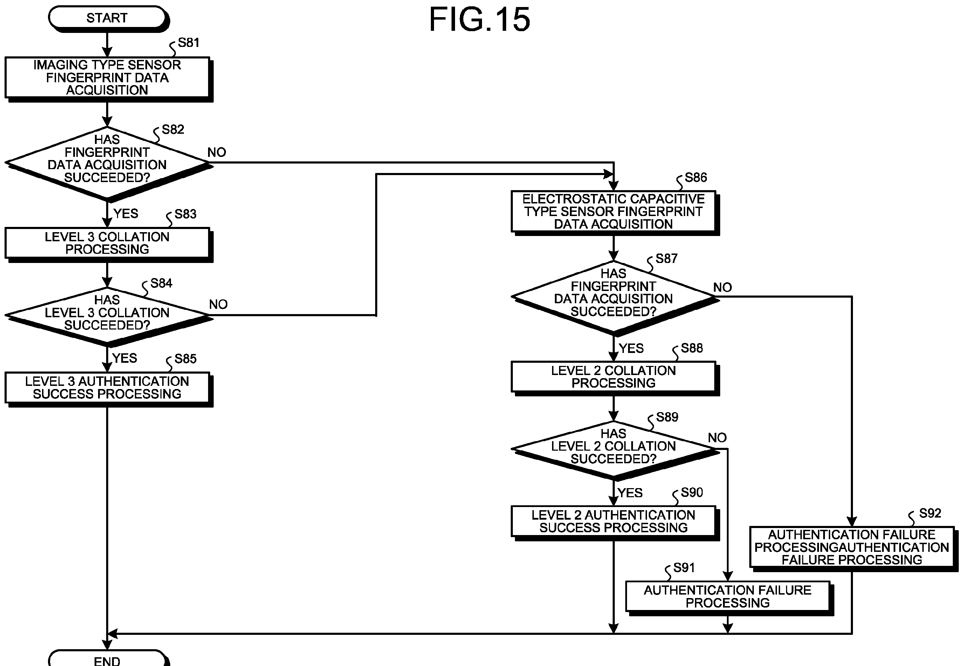

FIG. 15 is a processing flowchart of the first variation of the third embodiment.

First, the calculation unit 63 acquires the second fingerprint data from the second sensor 12 of an imaging type (Step S81).

Subsequently, the calculation unit 63 determines whether or not the second sensor 12 has succeeded in acquiring the second fingerprint data (Step S82).

When it is determined in Step S82 that the second sensor 12 has failed in acquiring the second fingerprint data (Step S82; No), the calculation unit 63 shifts the processing to Step S86.

When it is determined in Step S82 that the second sensor 12 has succeeded in acquiring the second fingerprint data (Step S82; Yes), the calculation unit 63 performs level 3 feature collation processing on the second fingerprint data acquired this time (Step S83).

Subsequently, the calculation unit 63 determines whether or not the level 3 feature collation processing in Step S83 has succeeded (Step S84).

When it is determined in Step S84 that the collation processing has succeeded (Step S84; Yes), the calculation unit 63 determines that the authentication has succeeded, performs level 3 authentication success processing such as shifting to processing after the fingerprint authentication as described above, and ends the processing (Step S85).

When it is determined in Step S84 that the collation processing has failed (Step S84; No), the calculation unit 63 acquires the first fingerprint data from the first sensor 11 of an electrostatic capacitive type (Step S86).

Subsequently, the calculation unit 63 determines whether or not the first sensor 11 has succeeded in acquiring the first fingerprint data (Step S87).

When it is determined in Step S87 that the first sensor 11 has failed in acquiring the first fingerprint data (Step S87; No), the calculation unit 63 performs acquisition failure processing such as notification of information indicating the failure in acquiring the fingerprint data, and ends the processing (Step S92).

When it is determined in Step S87 that the first sensor 11 has succeeded in acquiring the first fingerprint data (Step S87; Yes), the calculation unit 63 performs level 2 collation processing on the first fingerprint data acquired this time (Step S88).

Subsequently, the calculation unit 63 determines whether or not the level 2 feature collation processing in Step S88 has succeeded (Step S89).

When it is determined in Step S89 that the collation processing has succeeded (Step S89; Yes), the calculation unit 63 determines that the authentication has succeeded, performs level 2 authentication success processing such as shifting to processing after the fingerprint authentication as described above, and ends the processing (Step S90).

When it is determined in Step S89 that the collation processing has failed (Step S89; No), the calculation unit 63 determines that the authentication has failed, performs authentication failure processing such as notification of the information of the failure, and ends the processing (Step S91).

### Second Variation of Third Embodiment

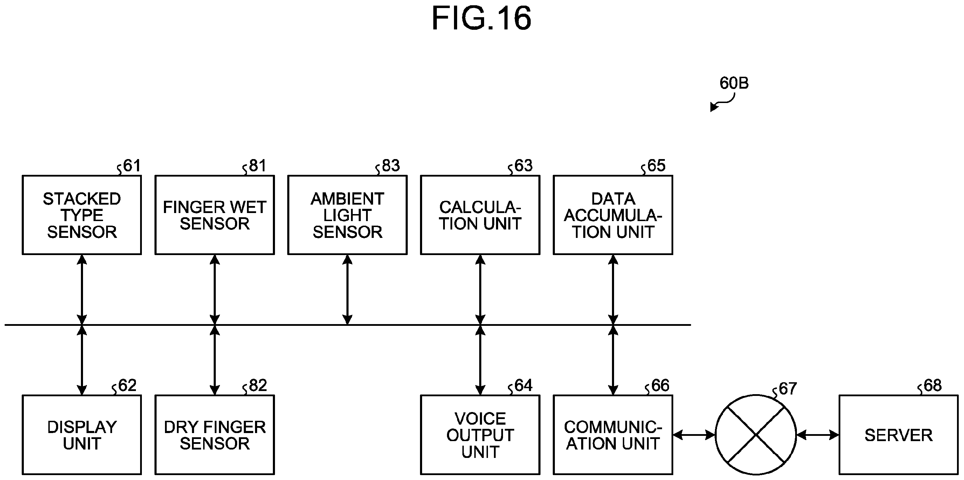

FIG. 16 is an explanatory diagram of a schematic system configuration example in the second variation of the third embodiment.

In FIG. 16, the same reference signs are attached to parts similar to those in FIG. 7.

The embodiment in FIG. 16 differs from the embodiment in FIG. 7 in that a finger wet sensor 81, a dry finger sensor 82, and an ambient light sensor 83 are provided. The finger wet sensor 81 detects whether or not a finger of a user is wet. The dry finger sensor 82 detects whether or not the finger of the user is dried. The ambient light sensor 83 detects a level of ambient light.

Incidentally, the second sensor 12 of an imaging type consumes a large amount of power, so that it is desirable that a sensor type can be preliminarily determined without activating the second sensor 12.

Therefore, in the second variation of the third embodiment, a sensor type to be used is preliminarily determined by providing the finger wet sensor 81, the dry finger sensor 82, and the ambient light sensor 83.

Next, operation in the second variation of the third embodiment will be described.

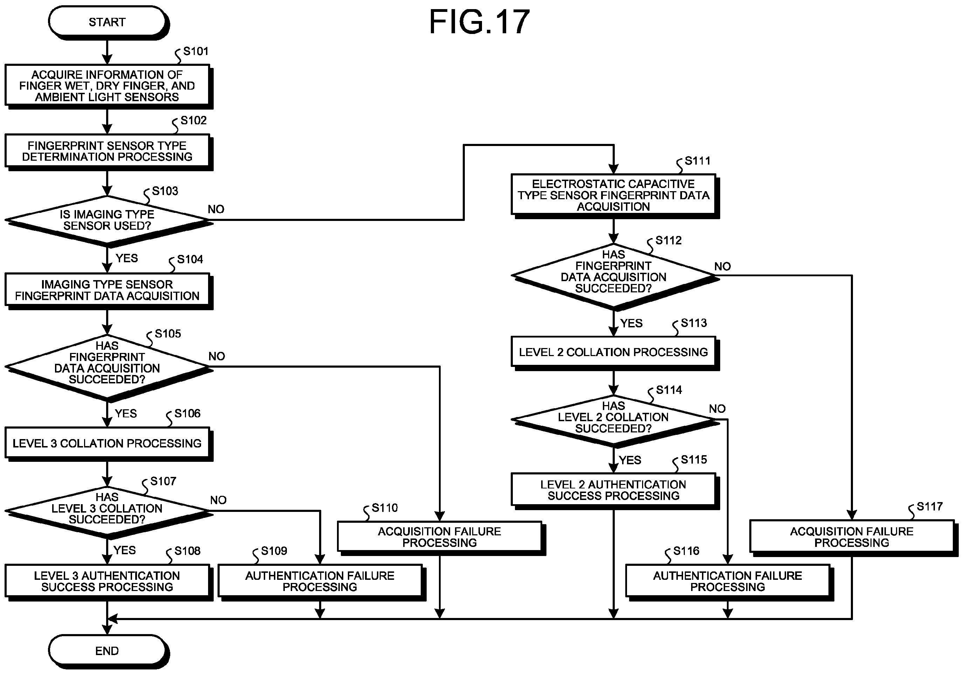

FIG. 17 is a processing flowchart of the second variation of the third embodiment.

First, the calculation unit 63 acquires information on the finger wet sensor 81, the dry finger sensor 82, and the ambient light sensor 83 (Step S101).

Subsequently, the calculation unit 63 performs processing of determining a fingerprint sensor type (Step S102).

In this case, the first sensor 11 of an electrostatic capacitive type has a relatively low finger wet resistance, and the second sensor 12 of an imaging type has a relatively high finger wet resistance. The first sensor 11 of an electrostatic capacitive type has a relatively high dry finger resistance, and the second sensor 12 of an imaging type has a relatively low dry finger resistance. The first sensor 11 of an electrostatic capacitive type receives a relatively little influence from ambient light (outside light), and the second sensor 12 of an imaging type receives a relatively large influence from the ambient light (outside light).

Therefore, the first sensor 11 of an electrostatic capacitive type is to be preferentially used for a dry finger and ambient light. The second sensor 12 of an imaging type is to be preferentially used for a finger wet. The calculation unit 63 determines a fingerprint sensor type to be used based on the information on the finger wet sensor 81, the dry finger sensor 82, and the ambient light sensor 83 and the above-described condition.

Next, the calculation unit 63 determines whether or not the second sensor 12 of an imaging type has been determined to be used (Step S103).

When it is determined in Step S103 that the second sensor 12 of an imaging type is determined to be used, the calculation unit 63 acquires the second fingerprint data with the second sensor 12 of an imaging type (Step S104).

Subsequently, the calculation unit 63 determines whether or not the second sensor 12 has succeeded in acquiring the second fingerprint data (Step S105).

When it is determined in Step S105 that the second sensor 12 has failed in acquiring the second fingerprint data (Step S105; No), the calculation unit 63 performs acquisition failure processing such as notification of information indicating the failure in acquiring the fingerprint data, and ends the processing (Step S110).

When it is determined in Step S105 that the second sensor 12 has succeeded in acquiring the second fingerprint data (Step S105; Yes), the calculation unit 63 performs the level 3 feature collation processing on the second fingerprint data acquired this time (Step S106) .

Subsequently, the calculation unit 63 determines whether or not the level 3 feature collation processing in Step S106 has succeeded (Step S107).

When it is determined in Step S107 that the collation processing has succeeded (Step S107; Yes), the calculation unit 63 determines that the authentication has succeeded, performs the level 3 authentication success processing such as shifting to processing after the fingerprint authentication as described above, and ends the processing (Step S108).

When it is determined in Step S107 that the collation processing has failed (Step S107; No), the calculation unit 63 determines that the authentication has failed, performs authentication failure processing such as notification of the information of the failure, and ends the processing (Step S109).

In contrast, when it is determined in Step S103 that the first sensor 11 of an electrostatic capacitive type is determined to be used, the calculation unit 63 acquires the first fingerprint data with the first sensor 11 of an electrostatic capacitive type (Step S111).

Subsequently, the calculation unit 63 determines whether or not the first sensor 11 has succeeded in acquiring the first fingerprint data (Step S112).

When it is determined in Step S112 that the first sensor 11 has failed in acquiring the first fingerprint data (Step S112; No), the calculation unit 63 performs acquisition failure processing such as notification of information indicating the failure in acquiring the fingerprint data, and ends the processing (Step S117).

When it is determined in Step S112 that the first sensor 11 has succeeded in acquiring the first fingerprint data (Step S112; Yes), the calculation unit 63 performs the level 2 collation processing on the first fingerprint data acquired this time (Step S113) .

Subsequently, the calculation unit 63 determines whether or not the level 2 feature collation processing in Step S88 has succeeded (Step S114).

When it is determined in Step S114 that the collation processing has succeeded (Step S114; Yes), the calculation unit 63 determines that the authentication has succeeded, performs the level 2 authentication success processing such as shifting to processing after the fingerprint authentication as described above, and ends the processing (Step S115).

When it is determined in Step S114 that the collation processing has failed (Step S114; No), the calculation unit 63 determines that the authentication has failed, performs authentication failure processing such as notification of the information of the failure, and ends the processing (Step S116).

As described above, according to the second variation of the third embodiment, a sensor to be used for fingerprint authentication is more suitably selected and activated depending on the state of a wet finger of a user, the state of a dry finger of the user, and a level of ambient light. This can reduce the failure-to-acquire rate (FTA) and power consumption, so that a prolonged period of use is made possible in, particularly, a wearable device.

### [4] Fourth Embodiment

In a fourth embodiment, the first sensor 11 of an electrostatic capacitive type, which operates with low power consumption, is used as a fingerprint authentication trigger, and a power supply of the second sensor 12 of an imaging type with a relatively high power consumption is controlled.

Incidentally, in order to achieve authentication with the level 3 feature amount, the second sensor 12 of an imaging type is required to acquire data.

The second sensor 12 of an imaging type, however, includes, for example, a CMOS sensor, and consumes a large amount of power. The second sensor 12 is desirably kept inactive except when necessary.

Therefore, in the fourth embodiment, the first sensor 11 of an electrostatic capacitive type, which operates with low power consumption, detects the state of a finger of a user, and a period during which the second sensor 12 of an imaging type is active is minimized. This allows reduction in power consumption as compared with a system that achieves the level 3 authentication with the second sensor 12 of an imaging type alone.

Since a device and a system configuration applied to the fourth embodiment are similar to those of the first embodiment, those will be referred to. Fingerprint authentication operation of the fourth embodiment will be described below on the assumption that fingerprint authentication data has already been registered.

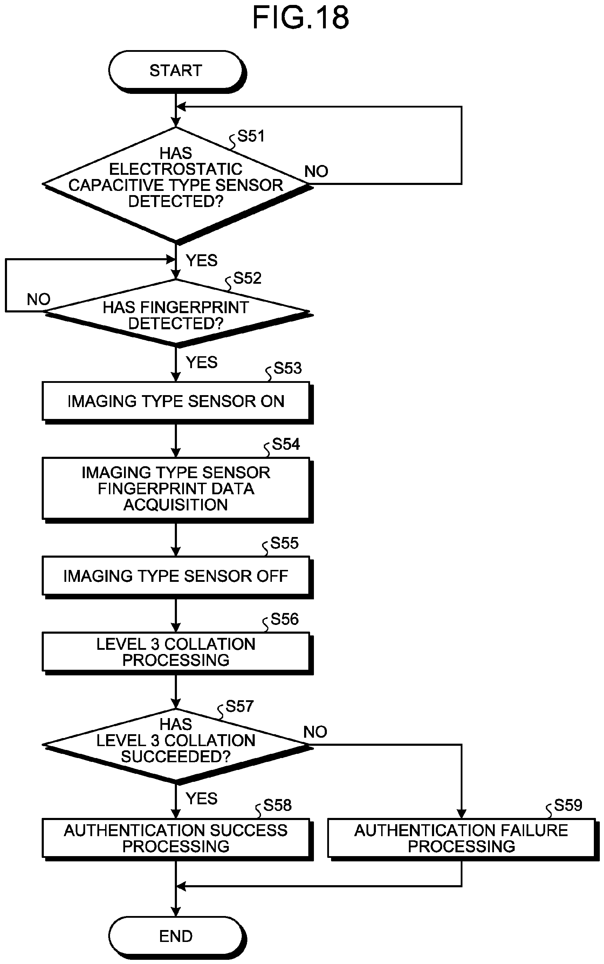

FIG. 18 is a processing flowchart of the fourth embodiment.

First, the calculation unit 63 determines whether or not the first sensor 11 of an electrostatic capacitive type has been detected to have been operated (Step S51).

When it is determined in Step S51 that the first sensor 11 of an electrostatic capacitive type has not been operated yet (Step S51; No), the calculation unit 63 enters a standby state.

When it is determined in Step S51 that the first sensor 11 of an electrostatic capacitive type has been operated (Step S51; Yes), the calculation unit 63 determines whether or not the first sensor 11 of an electrostatic capacitive type has detected a fingerprint (Step S52) .

When it is determined in Step S52 that a fingerprint has not been detected yet (Step S52; No), the calculation unit 63 enters a standby state.

When it is determined in Step S52 that a fingerprint has been detected (Step S52; Yes), the second sensor 12 of an imaging type is put in an ON state (driven state) (Step S53).

Then, the calculation unit 63 acquires the second fingerprint data from the second sensor 12 of an imaging type (Step S54).

Subsequently, the calculation unit 63 puts the second sensor 12 of an imaging type in an OFF state (non-driven state) (Step S55) .

Next, the calculation unit 63 performs the level 3 feature collation processing on the second fingerprint data acquired this time (Step S56).

Subsequently, the calculation unit 63 determines whether or not the level 3 feature collation processing in Step S56 has succeeded (Step S57).

When it is determined in Step S57 that the collation processing has succeeded (Step S57; Yes), the calculation unit 63 determines that the authentication has succeeded, performs the level 3 authentication success processing such as shifting to processing after the fingerprint authentication as described above, and ends the processing (Step S58).

When it is determined in Step S57 that the collation processing has failed (including when the second fingerprint data has failed to be acquired in Step S54) (Step S57; No), the calculation unit 63 determines that the authentication has failed, performs authentication failure processing such as notification of the information of the failure, and ends the processing (Step S59).

As described above, according to the fourth embodiment, power is supplied to the second sensor 12 of an imaging type having a relatively large power consumption to put the second sensor 12 in a driven state only when the first sensor 11 of an electrostatic capacitive type is operated and during a period when the second fingerprint data is acquired. The total power consumption can thus be reduced, and the time when, particularly, a wearable device, a mobile device, and the like can be used can be further prolonged.

### First Variation of Fourth Embodiment

Although, in the above-described fourth embodiment, the second sensor 12 of an imaging type is put in the driven state only when the first sensor 11 of an electrostatic capacitive type is operated and a fingerprint is detected, in the first variation of the fourth embodiment, power consumption is reduced by adaptively switching the first sensor 11 of an electrostatic capacitive type and the second sensor 12 of an imaging type in accordance with a required authentication level.

Next, operation in the first variation of the fourth embodiment will be described.

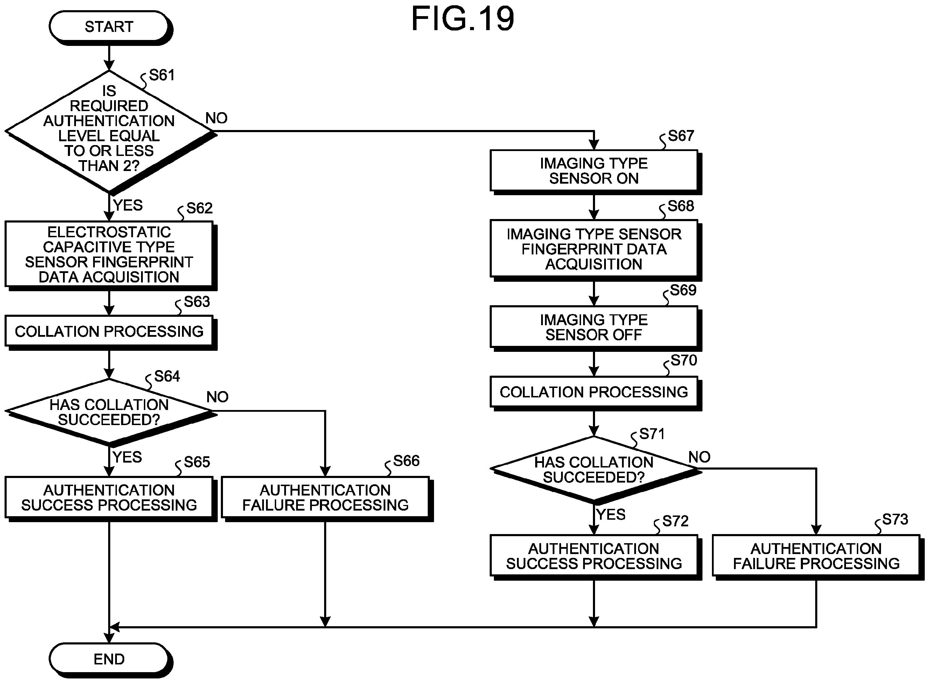

FIG. 19 is a processing flowchart of the first variation of the fourth embodiment.

First, the calculation unit 63 determines whether or not the required authentication level is equal to or less than the level 2 feature (Step S61).

When it is determined in Step S61 that the required authentication level is equal to or less than the level 2 feature (Step S61; Yes), the first sensor 11 of an electrostatic capacitive type acquires the first fingerprint data (Step S62).

Subsequently, the calculation unit 63 performs the collation processing on the first fingerprint data acquired this time (Step S63).

Subsequently, the calculation unit 63 determines whether or not the collation processing in Step S63 has succeeded (Step S64).

When it is determined in Step S64 that the collation processing has succeeded (Step S64; Yes), the calculation unit 63 determines that the authentication has succeeded, performs the authentication success processing such as shifting to processing after the fingerprint authentication as described above, and ends the processing (Step S65).

When it is determined in Step S64 that the collation processing has failed (including when the first fingerprint data has failed to be acquired in Step S62) (Step S64; No), the calculation unit 63 determines that the authentication has failed, performs authentication failure processing such as notification of the information of the failure, and ends the processing (Step S66).

In contrast, when it is determined in Step S61 that the required authentication level exceeds the level 2 feature, that is, when the required authentication level is the level 3 feature (Step S61; No), the second sensor 12 of an imaging type is put in an ON state (driven state) (Step S67).

Then, the calculation unit 63 acquires the second fingerprint data from the second sensor 12 of an imaging type (Step S68).

Subsequently, the calculation unit 63 puts the second sensor 12 of an imaging type in an OFF state (non-driven state) (Step S69).

Next, the calculation unit 63 performs the level 3 feature collation processing on the second fingerprint data acquired this time (Step S70).

Subsequently, the calculation unit 63 determines whether or not the level 3 feature collation processing in Step S56 has succeeded (Step S71).

When it is determined in Step S71 that the collation processing has succeeded (Step S71; Yes), the calculation unit 63 determines that the authentication has succeeded, performs the authentication success processing such as shifting to processing after the fingerprint authentication as described above, and ends the processing (Step S72).

When it is determined in Step S71 that the collation processing has failed (including when the second fingerprint data has failed to be acquired in Step S68) (Step S71; No), the calculation unit 63 determines that the authentication has failed, performs authentication failure processing such as notification of the information of the failure, and ends the processing (Step S73).

As described above, according to the first variation of the fourth embodiment, an optimal sensor can be driven in accordance with a required authentication level, so that power consumption can be further reduced, and the time when a wearable device or a mobile device can be driven can be prolonged.

### [5] Variations of Embodiment

### First Variation

Although each of the above-described embodiments has been described by using, as an example, the electronic device 50 configured as a wristband type wearable device, each of the embodiments can be applied to a smartphone, a stationary electronic device, a personal computer (e.g., sensing device 10 is provided in input device such as touch panel type display screen, keyboard, and mouse), a device mounted in a vehicle and the like (e.g., sensing device 10 is provided so as to be adjacent to touch panel type display screen or steering wheel), a head mount display (HMD) (e.g., sensing device 10 is provided in housing), an augmented reality (AR) glass (e.g., sensing device 10 is provided in frame part), and the like.

### Second Variation

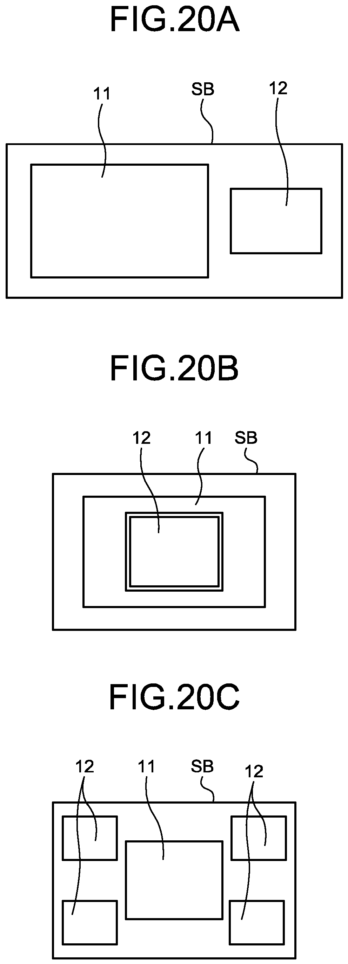

FIGS. 20A to 20C are explanatory views of variations of the embodiment.

FIG. 20A is an explanatory view of a second variation of the embodiment.

Although, in the above-described embodiments, the first sensor 11 of an electrostatic capacitive type and the second sensor 12 of an imaging type are stacked to form a stacked type sensor, in the second variation, the first sensor 11 of an electrostatic capacitive type and the second sensor 12 of an imaging type are arranged on a substrate SB in a known arrangement relation to form a sensing device.

Application similar to each of the above-described embodiments can be possible even if the first sensor 11 of an electrostatic capacitive type and the second sensor 12 of an imaging type are spaced apart from each other.

### Third Variation

FIG. 20B is an explanatory view of a third variation of the embodiment.

The third variation is an embodiment in which both the first sensor 11 of an electrostatic capacitive type with an opening and the second sensor 12 of an imaging type arranged in the opening are arranged on the same plane.

According to the third variation, the installation area of a sensor can be reduced, and the third variation can also be applied to a small device.

### Fourth Variation

FIG. 20C is an explanatory diagram of a fourth variation of the embodiment.

The fourth variation is an embodiment in which both the first sensor 11 of an electrostatic capacitive type and a plurality of second sensors 12 of an imaging type are arranged on the same plane. The first sensor 11 is arranged at the center on the substrate SB. The second sensors 12 are arranged around the first sensor 11.

According to the fourth variation, the plurality of second sensors 12 with a narrow sensing range is arranged, so that the sensing range of the second fingerprint data can be effectively increased, and the number of acquiring fingerprint data at the time of registering authentication data can be inhibited.

### Fifth Variation

Although, in the above description, a sensor of an electrostatic capacitive type is used as the first sensor 11, other types of sensors such as an ultrasonic sensor can be used. Note that, when a stacked type sensing device is configured, the other types of sensors are also required to have transparency.

### Sixth Variation

A sensing device of the present embodiment includes a control device, a storage device, a display device, and an input device. The control device includes a CPU and the like. The storage device includes a read only memory (ROM), a RAM, and the like. The display device includes a display and the like. The sensing device has hardware configuration using a common computer.

A program to be executed by the sensing device of the present embodiment is provided after being recorded in a computer-readable recording medium, such as a semiconductor memory device including a digital versatile disk (DVD), a USB memory, a solid state drive (SSD) and the like, in a file in an installable format or an executable format.

Furthermore, the program to be executed by the sensing device of the present embodiment may be provided by being stored on a computer connected to a network such as the Internet and downloaded via the network. Furthermore, the program to be executed by the sensing device of the present embodiment may be provided or distributed via a network such as the Internet.

Furthermore, the program of the sensing device of the present embodiment may be provided by being preliminarily incorporated in a ROM or the like.

Note that the embodiments of the present technology are not limited to the above-described embodiments, and various modifications can be made without departing from the gist of the present technology.

Moreover, the present technology can have the following configurations.
(1) A sensing device comprising:
   a first sensor that acquires three-dimensional shape information on a surface of an object to be sensed; and
   a second sensor that acquires texture information on the surface or a vicinity of the surface of the object to be sensed at a position having a predetermined positional relation with an information acquisition position in the object to be sensed of the first sensor.
(2) The sensing device according to (1),
   wherein the first sensor is configured as a sensor of an electrostatic capacitive type having transparency of detection light used by the second sensor, and
   the second sensor is configured as a sensor of an imaging type having a close-up lens.
(3) The sensing device according to (1) or (2),
   wherein the first sensor is configured as a fingerprint sensor.
(4) The sensing device according to any one of (1) to (3),
   wherein the first sensor and the second sensor are arranged in a stacked manner.
(5) The sensing device according to any one of (1) to (3),
   wherein the first sensor is arranged adjacently around the second sensor.
(6) The sensing device according to any one of (1) to (3),
   wherein the first sensor and the second sensor have a detection surface on a same plane, and are adjacently arranged at the position having a predetermined positional relation.
(7) The sensing device according to any one of (1) to (6), further comprising
   a calculation unit that extracts a feature amount of the object to be sensed and performs collation of the object to be sensed based on outputs of the first sensor and the second sensor.
(8) The sensing device according to (7), further comprising
   a data accumulation unit that accumulates registration data for identifying the object to be sensed,
   wherein the calculation unit performs collation of the object to be sensed based on outputs of the first sensor and the second sensor and the registration data.
(9) The sensing device according to (1),
   wherein the object to be sensed is a surface of each part of a living body such as a finger and a hand or a tissue in a vicinity of a surface of a living body such as a dermis and a blood vessel.
(10) The sensing device according to (1),
   wherein the three-dimensional shape information relates to, for example, a fingerprint.
(11) The sensing device according to (1),
   wherein the texture information includes information on, for example, a visual color and luminance, a pattern, and arrangement of a tissue in a vicinity of a surface, such as a surface shape of a surface or a dermis of each part of a human body or a living body and a flow of a blood vessel.
(12) The sensing device according to (1),
   wherein detection light used by the second sensor includes at least any one of visible light and invisible light such as an infrared light and an ultraviolet light.
(13) A method to be executed by an authentication device including: a first sensor that acquires three-dimensional shape information on a surface of an object to be sensed and outputs first data; a second sensor that acquires texture information on the surface of the object to be sensed at a position having a predetermined positional relation with an information acquisition position in the object to be sensed of the first sensor and outputs second data; and a calculation unit that extracts a feature amount of the object to be sensed and performs collation of the object to be sensed based on the first data and the second data, the method comprising the steps of:
   inputting a collation level; and
   performing the collation by using any one of the first data and the second data based on the collation level that has been input.
(14) The method according to (13),
   wherein the object to be sensed is wet or dried.
(15) A program for controlling, by using a computer, an authentication device including: a first sensor that acquires three-dimensional shape information on a surface of an object to be sensed and outputs first data; and a second sensor that acquires texture information on the surface or a vicinity of the surface of the object to be sensed at a position having a predetermined positional relation with an information acquisition position in the object to be sensed of the first sensor and outputs second data, the program causing the computer to function as:
   a determination unit that determines a collation level that has been input; and
   a collation unit that performs collation of the object to be sensed by using any one of the first data and the second data based on the collation level that has been determined.

### Reference Signs List

- 10: SENSING DEVICE
- 11: FIRST SENSOR
- 12: SECOND SENSOR
- 50: ELECTRONIC DEVICE
- 51: WRISTBAND MAIN BODY
- 52: SENSING DEVICE
- 53: DISPLAY UNIT
- 60: SYSTEM
- 61: STACKED TYPE SENSOR
- 62: DISPLAY UNIT
- 63: CALCULATION UNIT
- 64: VOICE OUTPUT UNIT
- 65: DATA ACCUMULATION UNIT
- 66: COMMUNICATION UNIT
- 67: COMMUNICATION NETWORK
- 68: SERVER
- 70: HIERARCHICAL REGISTERED FINGERPRINT DATABASE
- 71: ROUTE
- 72: RIDGE SHAPE DATA
- 73: FEATURE AMOUNT DATA
- 81: SENSOR
- 82: DRY FINGER SENSOR
- 83: AMBIENT LIGHT SENSOR
- SB: SUBSTRATE

## Claims

1. A sensing device comprising:
a first sensor that acquires three-dimensional shape information on a surface of an object to be sensed; and
a second sensor that acquires texture information on the surface or a vicinity of the surface of the object to be sensed at a position having a predetermined positional relation with an information acquisition position in the object to be sensed of the first sensor.

2. The sensing device according to claim 1,
wherein the first sensor is configured as a sensor of an electrostatic capacitive type having transparency of detection light used by the second sensor, and
the second sensor is configured as a sensor of an imaging type having a close-up lens.

3. The sensing device according to claim 1,
wherein the first sensor is configured as a fingerprint sensor.

4. The sensing device according to claim 1,
wherein the first sensor and the second sensor are arranged in a stacked manner.

5. The sensing device according to claim 1,
wherein the first sensor is arranged adjacently around the second sensor.

6. The sensing device according to claim 1,
wherein the first sensor and the second sensor have a detection surface on a same plane, and are adjacently arranged at the position having a predetermined positional relation.

7. The sensing device according to claim 1, further comprising
a calculation unit that extracts a feature amount of the object to be sensed and performs collation of the object to be sensed based on outputs of the first sensor and the second sensor.

8. The sensing device according to claim 7, further comprising
a data accumulation unit that accumulates registration data for identifying the object to be sensed,
wherein the calculation unit performs collation of the object to be sensed based on outputs of the first sensor and the second sensor and the registration data.

9. The sensing device according to claim 1,
wherein the object to be sensed is a surface of each part of a living body such as a finger and a hand or a tissue in a vicinity of a surface of a living body such as a dermis and a blood vessel.

10. The sensing device according to claim 1,
wherein the three-dimensional shape information relates to, for example, a fingerprint.

11. The sensing device according to claim 1,
wherein the texture information includes information on, for example, a visual color and luminance, a pattern, and arrangement of a tissue in a vicinity of a surface, such as a surface shape of a surface or a dermis of each part of a human body or a living body and a flow of a blood vessel.

12. The sensing device according to claim 1,
wherein detection light used by the second sensor includes at least any one of visible light and invisible light such as an infrared light and an ultraviolet light.

13. A method to be executed by an authentication device including: a first sensor that acquires three-dimensional shape information on a surface of an object to be sensed and outputs first data; a second sensor that acquires texture information on the surface of the object to be sensed at a position having a predetermined positional relation with an information acquisition position in the object to be sensed of the first sensor and outputs second data; and a calculation unit that extracts a feature amount of the object to be sensed and performs collation of the object to be sensed based on the first data and the second data, the method comprising the steps of:
inputting a collation level; and
performing the collation by using any one of the first data and the second data based on the collation level that has been input.

14. The method according to claim 13,
wherein the object to be sensed is wet or dried.

15. A program for controlling, by using a computer, an authentication device including: a first sensor that acquires three-dimensional shape information on a surface of an object to be sensed and outputs first data; and a second sensor that acquires texture information on the surface or a vicinity of the surface of the object to be sensed at a position having a predetermined positional relation with an information acquisition position in the object to be sensed of the first sensor and outputs second data, the program causing the computer to function as:
a determination unit that determines a collation level that has been input; and
a collation unit that performs collation of the object to be sensed by using any one of the first data and the second data based on the collation level that has been determined.
